# EUROPEAN PATENT APPLICATION

(11) **EP 1 671 632 A1**
(43) Date of publication of application: **21.06.2006**
(21) Application number: 06000777.0
(22) Date of filing: 16.08.2001
(51) Int. Cl.: A61K 31/403, A61K 31/4184, A61K 45/06, A61P 9/10, A61P 9/12, A61P 11/00, A61P 35/00

(54) **Pharmaceutical combination of angiotensin II antagonists and ACE inhibitors**

(30) Priority: 22.08.2000 GB 0020691; 20.02.2001 DE 10108215
(62) Divisional of application: 01976082.6
(71) Applicant: Boehringer Ingelheim Pharma GmbH & Co. KG, 55216 Ingelheim (DE)
(72) Inventor: Böhm, Peter, 55543 Bad Kreuznach (DE); Meinicke, Wolf Thomas, 88441 Mittelbiberach (DE); Riedel, Axel, 88437 Maselheim (DE)
(74) Representative: Hammann, Heinz

(57) **Abstract**

The invention relates to a method of treatment of indications which can be positively influenced by inhibition of AT₁ mediated effects with maintenance of AT₂ receptor mediated effects of Angiotensin II and by ACE inhibition, thus also increasing bradykinin mediated effects, e.g. to reduce the incidence of stroke, acute myocardial infarction or cardiovascular death, or of indications associated with the increase of AT₁ receptors in the sub-epithelial area or increase of AT₂ receptors in the epithelia, comprising co-administration of effective amounts of an Angiotensin II antagonist and an ACE inhibitor, pharmaceutical compositions containing an Angiotensin II antagonist together with an ACE inhibitor and the use of an Angiotensin II antagonist and an ACE inhibitor for the manufacture of corresponding pharmaceutical compositions.

## Description

### Field of the Invention

This invention relates to a method of treatment of **indications (A)** which can be positively influenced by inhibition of AT₁ mediated effects with maintenance of AT₂ receptor mediated effects of Angiotensin II (ANG II) and
by ACE inhibition, thus also increasing bradykinin mediated effects,
e.g. to reduce the incidence of stroke, acute myocardial infarction or cardiovascular death, especially in persons having elevated risk of cardiovascular events or stroke,
or of **indications (B)** associated with the increase of AT₁ receptors in the sub-epithelial area or increase of AT₂ receptors in the epithelia,
which method comprises co-administration of effective amounts of an ANG II antagonist and an ACE inhibitor to a person in need of such treatment,
suitable pharmaceutical compositions comprising an ANG II antagonist and an ACE inhibitor as a combined preparation for simultaneous, separate or sequential use in treatment of said indications and
the use of an ANG II antagonist for manufacture of a pharmaceutical composition for treatment of said indications when used in combination with an ACE inhibitor.

The beneficial efficacy of the methods according to the invention seems to be mainly based on organoprotective, tissue-protective and vasculoprotective effects of the combined treatment.

### Background of the Invention

ANG II plays a major role in pathophysiology, especially as the most potent blood pressure increasing agent in humans. ANG II antagonists therefore are suitable for treating elevated blood pressure and congestive heart failure in a mammal. Examples of ANG II antagonists are described in EP-A-0 502 314, EP-A-0 253 310 , EP-A-0 323 841, EP-A-0 324 377, US-A-4,355,040 and US-A-4,880,804 . Specific ANG II antagonists are sartans such as candesartan, eprosartan, irbesartan, losartan, telmisartan or valsartan, furthermore olmesartan and tasosartan.

A series of angiotensin I converting enzyme (ACE) inhibitors also are known as antihypertensives and for treatment of congestive heart failure, e.g. benazepril, captopril, ceronapril, enalapril, fosinopril, imidapril, lisinopril, moexipril, quinapril, ramipril, trandolapril, perindopril. Examples are described in EP-A-0 079 022 , US-A-4,046,889 and 4,374,829.

It is known that ANG II, besides its blood pressure increasing effect, additionally features growth promoting effects contributing to left ventricular hypertrophy, vascular thickening, atherosclerosis, renal failure and stroke. Bradykinin, on the other hand, exerts vasodilating and tissue protective actions, as disclosed in the following publications:

W Wienen et al.: Antihypertensive and renoprotective effects of telmisartan after long term treatment in hypertensive diabetic (D) rats, 2^{nd}. Int. Symposium on Angiotensin II Antagonism, February 15-18, 1999, The Queen Elizabeth II Conference Center, London, UK, Book of Abstracts, Abstract No. 50, J Wagner et al.: Effects of AT₁ receptor blockade on blood pressure and the renin angiotensin system in spontaneously hypertensive rats of the stroke prone strain, Clin Exp Hypertens 1998, 20: 205-221, and

M Bohm. et al.: Angiotensin II receptor blockade in TGR(mREN2)27: effects of renin-angiotensin-system gene expression and cardiovascular functions, J Hypertens 1995, 13 8: 891-899.

Losartan and irbesartan provide a renoprotective effect found within first clinical trials, as disclosed in the following publications:

S Andersen et al.: Renoprotective effects of angiotensin II receptor blockade in type 1 diabetic patients with diabetic nephropathy, Kidney Int 57 (2), 601-606 (2000),

LM Ruilope: Renoprotection and renin-angiotensin system blockade in diabetes mellitus, Am J Hypertens 10(12 PT 2) Suppl), 325S-331S (1997),

JFE Mann: Valsartan and the kidney: Present and future, J Cardiovasc Pharmacol 33 Suppl 1, S37-S40 (1999),

EL Schiffrin et al.: Correction of arterial structure and endothelial dysfunction in human essential hypertension by the angiotensin receptor antagonist losartan, Circulation 101(14), 1653-1659 (2000),

RM Touyz et al.: Angiotensin II stimulates DNA and protein synthesis in vascular smooth muscle cells from human arteries: role of extracellular signal-regulated kinases, J Hypertens 17(7), 907-916 (1999),

EL Schiffrin: Vascular remodeling and endothelial function in hypertensive patients: Effects of antihypertensive therapy, Scand Cardiovasc J 32 Suppl 47, 15-21 (1998) and

A Prasad: Acute and chronic angiotensin-1 receptor antagonism reverses endothelial dysfunction in atherosclerosis, Circulation 2000; 101: 2349 cont.

Furthermore it has been found that the antiproteinuric effect of enalapril is potentiated by losartan in normotensive patients with diabetic nephropathy, as published in Am J Hypertens 13 (4, Part 2), 117A Abstr A017 (2000) referring to the 15th Sci Mtg of the American Society of Hypertension, 16 - 20 May 2000.

Results of the Heart Outcomes Prevention Evaluation (HOPE) study (New Engl J Med 432/3, January 20, 2000, p 145-153) indicate that treatment with ACE inhibitor ramipril significantly reduces the risk of the combined primary cardiovascular outcome by 22% and consistently reduces the risk for secondary endpoints of outcome, including total mortality. The cardiovascular benefit was shown to be largely independent from the blood pressure lowering effect suggesting that ramipril exerts an independent vasculoprotective and organoprotective effect.

There is however also evidence that chronic treatment with ACE inhibitors does not suppress ANG II levels effectively due to compensatory activation of other ANG II-generating enzymes (e.g. human chymase, cathepsin G) which may have deleterious effects, particularly ongoing end organ damage, due to continued AT₁ receptor mediated action of ANG II (mechanisms described e.g. in review article of Willenheimer, Eur. Heart J. 1999; 20, 997 - 1008).

Ang II antagonists selectively block the AT₁ receptor, leaving the AT₂ receptor, which plays a role in anti-growth and tissue regenerative actions, unopposed. Completed clinical trials with Ang II antagonists appear to display similar blood pressure reducing and tissue protective effects as with ACE inhibitors, as disclosed in the following publications:
DHG Smith et al.: Once-daily telmisartan compared with enalapril in the treatment of hypertension, Adv. Ther 1998, 15: 229-240,
BE Karlberg et al.: Efficacy and safety of telmisartan, a selective AT1 receptor antagonist, compared with enalapril in elderly patients with primary hypertension, J Hypertens 1999, 17: 293-302, and
JM Neutel et al.: Comparison of telmisartan with lisinopril in patients with mild-to-moderate hypertension, Am J Ther 1999, 6, 161-166.

Most recently, attention has been focussed on the combination of both drug principles in the treatment of congestive heart failure based on the rationale to combine the benefits of ACE inhibition and bradykinin potentiation together with a more efficient inhibition of the Renin-Angiotensin-Aldosteron system via AT₁ receptor blockade and a shift of the actions of the remaining ANG II from the AT₁ to the AT₂ receptor (M Burnier, IDrugs 3 (3): 304-309, (2000)). Pharmacologically, this is a highly attractive approach, and large studies are currently ongoing in congestive heart failure (VAL-HeFT, Cardiology 1999, 91 (Suppl I), 19-22; CHARM, J Cardiac Failure 1999, 5: 276-282) to prove this hypothesis.

Combined treatment and corresponding compositions comprising amounts of at least two therapeutic agents selected from the group consisting of a renin inhibitor, an ACE inhibitor and an ANG II antagonist, in amounts sufficient to cause synergistic therapeutic effects in lowering blood pressure and treating congestive heart failure in a mammal are disclosed in EP-A-0 527 879 . Preferred ACE inhibitors are taught to be captopril, enalapril, lisinopril and ramipril. Losartan is disclosed as the preferred ANG II antagonist. Dosage ranges for ACE inhibitors are disclosed to include 40 mg/day to 450 mg/day orally and 20 mg/day parenterally. Dosage ranges for ANG II antagonists are disclosed to include 0.5 to 500 mg/kg p.o., preferably 2 to 80 mg/kg p.o., and 3 mg/kg i.v.

EP-A-1 013 273 discloses the use of AT₁ receptor antagonists or AT₂ receptor modulators for treating diseases associated with an increase of AT₁ receptors in subepithelial area or increase of AT₂ receptors in the epithelia, especially for treatment of several lung diseases.

### Summary of the Invention

It has been found that co-administration of an ANG II antagonist with an ACE inhibitor provides unexpected advantages in the treatment of **indications (A)** which can be positively influenced by inhibition of AT₁ mediated effects with maintenance of AT₂ receptor mediated effects of ANG II and
by ACE inhibition, thus also increasing bradykinin mediated effects,
furthermore in the treatment of **indications (B)** associated with the increase of AT₁ receptors in the sub-epithelial area or increase of AT₂ receptors in the epithelia,
with high efficacy, independently from the known blood pressure reducing activity of these agents, in comparison to administration of an ANG II antagonist or ACE inhibitor alone.

These indications are meant to include indications which can be positively influenced by the superior organoprotective, tissue-protective and vasculoprotective effects provided by the combined treatment using an ANG II antagonist together with an ACE inhibitor, e.g. **indications (A)** may include
such as reduction of the incidence of stroke, acute myocardial infarction or cardiovascular death, especially in persons having elevated risk of cardiovascular events or stroke,
renoprotection, e.g. in renal failure or diabetic nephropathy,
left ventricular hypertrophy, vascular thickening, e.g. prevention of thickening of blood vessel walls after vascular operations, prevention of arterial re-stenosis after angioplasty, prevention or treatment of atherosclerosis, prevention of diabetic angiopathy,
ischaemic peripheral circulatory disorders, myocardial ischaemia (angina), prevention of the progression of cardiac insufficiency after myocardial infarction,
**indications (B)** associated with the increase of AT₁ receptors in the sub-epithelial area or increase of AT₂ receptors in the epithelia may include
such as obstructive airways diseases, chronic obstructive pulmonary disease, e.g. bronchitis or chronic bronchitis, emphysema, likewise from asthma, cystic fibrosis, interstitial lung disease, lung cancer, pulmonary vascular disease, and increased resistance to airflow during forced expiration,
adults respiratory distress syndrome (ARDS), reducing the proliferative capacity of the epithelium in lung and breast cancer, the treatment of sepsis syndrome, lung injury forms, such as pneumonia aspiration of gastric content, chest trauma, shock, burns, fat embolia, cardiopulmonary bypass, O₂ toxicity, haemorhagic pancreatitis, interstitial and bronchoalveolar inflammation, proliferation of epithelial and interstitial cells, collagen accumulation or fibrosis.

The unexpected advantages mentioned above may be due to more efficient blockade of AT₁ mediated effects of ANG II and due to AT₂ receptor mediated action of ANG II, left unaffected by ANG II antagonists, together with an increase of bradykinin mediated effects caused by ACE inhibitors.

According to a first aspect the present invention provides a method of treatment of **indications (A)** which can be positively influenced by inhibition of AT₁ mediated effects with maintenance of AT₂ receptor mediated effects of ANG II and
by ACE inhibition, thus also increasing bradykinin mediated effects,
or of **indications (B)** associated with the increase of AT₁ receptors in the sub-epithelial area or increase of AT₂ receptors in the epithelia,
which method comprises co-administration of effective amounts of an ANG II antagonist and an ACE inhibitor to a human or non-human mammalian body in need of such treatment.

Details of the indications which can be treated using a method according to the invention are given hereinbefore.

It has been found that co-administration of ANG II antagonists with ACE inhibitors provides significant prevention of cardiovascular death and all-cause mortality, especially with regard to incidence of stroke and acute myocardial infarction, when compared to administration of an ANG II antagonist or ACE inhibitor alone.

Therefore a preferred method according to the present invention is to reduce incidence of stroke and acute myocardial infarction in the human or non-human mammalian body in need thereof, especially in persons having elevated risk of cardiovascular events or stroke, by co-administration of an ANG II antagonist with an ACE inhibitor.

Furthermore, it has been found that combined treatment and corresponding compositions specifically comprising an amount of the ACE inhibitor ramipril together with an amount of the ANG II antagonist telmisartan are highly active in lowering blood pressure and treating congestive heart failure in a mammal. It is expected that the synergistic effect provided by this specific combination is surprisingly superior over corresponding combinations known in the art. A synergistic combination according to the invention for lowering elevated blood pressure or treatment of congestive heart failure is meant to comprise an amount of ramipril and an amount of telmisartan wherein the amount of the individual agent alone is insufficient to achieve the therapeuticv effect achieved by the administration of the combination of said agents and wherein the combined effects of the amounts of the therapeutic agents is greater than the sum of the therapeutic effects achievable with the amounts of the individual therapeutic agents.

Viewed from a different aspect the present invention also relates to pharmaceutical compositions for the treatment of the human or non-human mammalian body for treating the indications mentioned hereinbefore comprising an ANG II antagonist and an ACE inhibitor, optionally together with pharmaceutically acceptable diluents and/or carriers, as a combined preparation for simultaneous, separate or sequential use in treatment of said indications.

Viewed from a further aspect the present invention provides the use of an ANG II antagonist for manufacture of a pharmaceutical composition for treatment of the indications mentioned hereinbefore when used in combination with an ACE inhibitor.

### Detailed Description of the Invention

With regard to all aspects of the invention any ANG II antagonist may be suitable, unless otherwise specified, e.g. the sartans such as candesartan, eprosartan, irbesartan, losartan, telmisartan, valsartan, ol-mesartan and tasosartan mentioned hereinbefore, preferably losartan or telmisartan, most preferred telmisartan {4'-[2-n-propyl-4-methyl-6-(1-methyl-benzimidazol-2-yl)benzimidazol-1-ylmethyl]biphenyl-2-carboxylic acid},
furthermore, any ACE inhibitor may be used with regard to all aspects of the invention mentioned hereinbefore, unless otherwise specified, e.g. benaz-epril, captopril, ceronapril, enalapril, fosinopril, imida-pril, lisinopril, moexipril, quinapril, ramipril, trandolapril and perindopril, preferably captopril, enalapril, lisinopril and ramipril, most preferred ramipril.

In a preferred embodiment of the method-of-treatment aspect ramipril is co-administered with any ANG II antagonist.

In a second preferred embodiment of the method-of-treatment aspect any ACE inhibitor is co-administered with telmisartan.

In a third preferred embodiment of the method-of-treatment aspect ramipril is co-administered with telmisartan.

Co-administration of an ANG II antagonist and an ACE inhibitor is meant to include administration sequential in time or simultaneous administration, the simultaneous administration being preferred. For sequential administration, the ANG II antagonist can be administered before or after administration of the ACE inhibitor.

The active compounds can be administered orally, bucally, parenterally, by inhalation spray, rectally or topically, the oral administration being preferred. Parenteral administration may include subcutaneous, intravenous, intramuscular and in-trasternal injections and infusion techniques.

The active compounds can be orally administered in a wide variety of different dosage forms, i.e., they may be formulated with various pharmaceutically acceptable inert carriers in the form of tablets, capsules, lozenges, troches, hard candies, powders, sprays, aqueous suspensions, elixirs, syrups, and the like. Such carriers include solid diluents or fillers, sterile aqueous media and various non-toxic organic solvents, etc. Moreover, such oral pharmaceutical formulations can be suitably sweetened and/or flavored by means of various agents of the type commonly employed for such purposes. In general, the compounds of this invention are present in such oral dosage forms at concentration levels ranging from about 0.5% to about 90% by weight of the total composition, in amounts which are sufficient to provide the desired unit dosages. Other suitable dosage forms for the compounds of this invention include controlled release formulations and devices well known to those who practice in the art.

For purposes of oral administration, tablets containing various excipients such as sodium citrate, calcium carbonate and calcium phosphate may be employed along with various disintegrants such as starch and preferably potato or tapioca starch, alginic acid and certain complex silicate, together with binding agents such as polyvinylpyrrolidone, sucrose, gelatin and acacia. Additionally, lubricating agents such as magnesium stearate, sodium lauryl sulfate and talc or compositions of a similar type may also be employed as fillers in soft and hard-filled gelatin capsules; included lactose or milk sugar as well as high molecular weight polyethylene glycols. When aqueous suspensions and/or elixirs are desired for oral administration, the essential active ingredient therein may be combined with various sweetening or flavoring agents, coloring matter or dyes and, if so desired, emulsifying agents and/or water, ethanol, propylene glycol, glycerin and various like combinations thereof.

For purposes of parenteral administration, solutions of the compounds in sesame or peanut oil or in aqueous propylene glycol may be employed, as well as sterile aqueous solutions of the corresponding pharmaceutically-acceptable salts. Such aqueous solutions should be suitably buffered if necessary, and the liquid diluent rendered isotonic with sufficient saline or glucose. These particular aqueous solutions are especially suitable for intravenous, intramuscular and subcutaneous injection purposes. In this connection, the sterile aqueous media employed are readily obtained by standard techniques well known to those skilled in the art. For instance, distilled water is ordinarily used as the liquid diluent and the final preparation is passed through a suitable bacterial filter such as a sintered glass filter or a diatomaceous-earth or unglazed porcelain filter. Preferred filters of this type include the Berkefeld, the Chamberland and the Asbestos Disk-Metal Seitz filter, wherein the fluid is sucked into a sterile container with the aid of a suction pump. The necessary steps should be taken throughout the preparation of these injectable solutions to insure that the final products are obtained in a sterile condition. For purposes of transdermal administration, the dosage form of the particular compound or compounds may include, by way of example, solutions, lotions, ointments, creams, gels, suppositories, rate-limiting sustained release formulations and devices therefor. Such dosage forms comprise the particular compound or compounds and may include ethanol, water, penetration enhancer and inert carriers such as gel-producing materials, mineral oil, emulsifying agents, benzyl alcohol and the like.

Several ANG II inhibitors are already on the market and can be used for administration, e.g. Micardis®, Lorzaar®, Cozaar®, Lortaan® Losaprex®, Neo-Lotan® or Oscaar®, Approvel®, Karvea®,

Diovan®, Atacand®, Blopress® and Teveten®.

Also several ACE-inhibitors are already on the market and can be used for administration, e.g. Briem®, Cibacen®, Cibacne®, Lotensin®, Dynacil®, Elidiur®, Fosinorm®, Fositen®, Fozitec®, Monopril®, Staril®, Tensozide®, Novaloc®, Tanapril®, Fempress®, Perdix®, Univasc®, Accupril®, Accuprin®, Accupro®, Acequin®, Acuitel®, Korec®, Quinazil®, Xanef®, Pres®, Acerbon®, Lopirin®, Tensobon®, Delix® or Vesdil®.

The ACE inhibitor may be administered in a daily dosage of 1.25 mg (or 0.018 mg/kg, based on a person of 70 kg) to 450 mg (0.571 mg/kg) orally and of about 20 mg (0.286 mg/kg) parenterally, preferably of 5 mg (0.071 mg/kg) to 100 mg (1.429 mg/kg) orally. Particularly preferred is an oral daily dosage of 5 (0.071 mg/kg) to 30 mg (0.429 mg/kg), or specifically of about 10 mg (0.143 mg/kg)

The ANG II antagonist may be administered in a daily dosage of 10 mg (or 0.143 mg/kg, based on a person of 70 kg) to 500 mg (7.143 mg/kg) orally and of about 20 mg (0.286 mg/kg) parenterally, preferably of 20 mg (0.286 mg/kg) to 100 mg (1.429 mg/kg) orally. Particularly preferred is an oral daily dosage of 40 mg (0.571 mg/kg) to 80 mg (1.143 mg/kg) or specifically of about 80 mg (1.143 mg/kg).

In all administration modes and dosages mentioned hereinbefore the preferred ACE inhibitor is ramipril and the preferred ANG II antagonist is telmisartan. In the most preferred embodiment ramipril is administered simultaneously in a daily dosage of about 10 mg together with telmisartan in a daily dosage of about 80 mg via the oral route.

The pharmaceutical compositions of this invention contain one ACE inhibitor in an amount of 1.25 mg to 450 mg and one ANG II antagonist in an amount of 10 mg to 500 mg in single dosage units, optionally together with one or more pharmaceutically acceptable diluents and/or carriers,
for instance the pharmaceutical compositions of this invention contain one ACE inhibitor selected from benazepril, captopril, ceronapril, enalapril, fosinopril, imidapril, lisinopril, mo-exipril, quinapril, ramipril, trandolapril and perindopril in an amount of 1.25 mg to 100 mg and one ANG II antagonist selected fom candesartan, eprosartan, irbesartan, losartan, telmisartan and valsartan, olmesartan, tasosartan in an amount of 20 to 100 mg in single dosage units, with exception of the combination of captopril with losartan, optionally together with one or more pharmaceutically acceptable diluents and/or carriers.

A preferred subgroup of pharmaceutical compositions of this invention contain as ACE inhibitor ramipril in an amount of 1.25 mg to 100 mg and one ANG II antagonist selected from candesartan, eprosartan, irbesartan, losartan, telmisartan and valsartan in an amount of 20 mg to 100 mg in single dosage units, optionally together with one or more pharmaceutically acceptable diluents and/or carriers.

A second preferred subgroup of pharmaceutical compositions of this invention contain one ACE inhibitor selected from benazepril, captopril, ceronapril, enalapril, fosinopril, imidapril, lisinopril, moexipril, quinapril, ramipril and trandolapril in an amount of 1.25 mg to 100 mg and as ANG II antagonist telmisartan in an amount of 20 mg to 100 mg in single dosage units, optionally together with one or more pharmaceutically acceptable diluents and/or carriers.

A third preferred subgroup of pharmaceutical compositions of this invention contain one ACE inhibitor selected from enalapril, lisinopril and ramipril in an amount of 1.25 mg to 100 mg and one ANG II antagonist selected fom losartan and telmisartan in an amount of 20 mg to 100 mg, in single dosage units, optionally together with one or more pharmaceutically acceptable diluents and/or carriers.

The most preferred pharmaceutical compositions of this invention contain as ACE inhibitor ramipril in an amount of 1.25 mg to 100 mg and as ANG II antagonist telmisartan in an amount of 20 mg to 100 mg, in single dosage units, optionally together with one or more pharmaceutically acceptable diluents and/or carriers.

Especially preferred pharmaceutical compositions of this invention contain as ACE inhibitor ramipril in an amount of about 10 mg and as ANG II antagonist telmisartan in an amount of about 80 mg in single dosage units, optionally together with one or more pharmaceutically acceptable diluents and/or carriers.

As already mentioned above the present invention also provides the use of an ANG II antagonist for manufacture of a pharmaceutical composition for the treatment of the human or non-human mammalian body for treating the indications mentioned hereinbefore when used in combination with an ACE inhibitor. This use aspect is meant to include the manufacture of all pharmaceutical compositions mentioned hereinbefore in accordance with the invention.

## Claims

1. A method of treatment of **indications (A)** which can be positively influenced by inhibition of AT₁ mediated effects with maintenance of AT₂ receptor mediated effects of Angiotensin II (ANG II) and
by ACE inhibition, thus also increasing bradykinin mediated effects,
or of **indications (B)** associated with the increase of AT₁ receptors in the sub-epithelial area or increase of AT₂ receptors in the epithelia,
which method comprises co-administration of effective amounts of an ANG II antagonist and an ACE inhibitor to a person in need of such treatment.

2. The method of claim 1, **characterized in that** the **indications (A)** are selected from
reduction of the incidence of stroke, acute myocardial infarction or cardiovascular death, especially in persons having elevated risk of cardiovascular events or stroke,
renoprotection, e.g. in renal failure or diabetic nephropathy,
left ventricular hypertrophy, vascular thickening, e.g. prevention of thickening of blood vessel walls after vascular operations, prevention of arterial re-stenosis after angioplasty, prevention or treatment of atherosclerosis, prevention of diabetic angiopathy,
ischaemic peripheral circulatory disorders, myocardial ischaemia (angina) and prevention of the progression of cardiac insufficiency after myocardial infarction.

3. The method of claim 1, **characterized in that** the **indications (B)** are selected from
obstructive airways diseases, chronic obstructive pulmonary disease, e.g. bronchitis or chronic bronchitis, emphysema, likewise from asthma, cystic fibrosis, interstitial lung disease, lung cancer, pulmonary vascular disease, and increased resistance to airflow during forced expiration,
adults respiratory distress syndrome (ARDS), reducing the proliferative capacity of the epithelium in lung and breast cancer, the treatment of sepsis syndrome, lung injury forms, such as pneumonia aspiration of gastric content, chest trauma, shock, burns, fat embolia, cardiopulmonary bypass, O₂ toxicity, haemorhagic pancreatitis, interstitial and bronchoalveolar inflammation, proliferation of epithelial and interstitial cells, collagen accumulation and fibrosis.

4. The method of any of claims 1 to 3, **characterized in that** the ACE inhibitor is selected from benazepril, captopril, ceronapril, enalapril, fosinopril, imidapril, lisinopril, moexipril, quinapril, ramipril, trandolapril and perindopril.

5. The method of any of claims 1 to 4, **characterized in that** the ANG II antagonist is selected from the sartans.

6. A method of treatment of elevated blood pressure or congestive heart failure in a mammal comprising co-administration of an effective amount of the ACE inhibitor ramipril together with an effective amount of the ANG II antagonist telmisartan.

7. The method of any of claims 1 to 6, **characterized in that** the ACE inhibitor is administered in a daily dosage of 0.018 mg/kg to 0.571 mg/kg orally or of about 0.286 mg/kg parenterally and the ANG II antagonist is administered in a daily dosage of 0.143 mg/kg to 7.143 mg/kg orally or of about 0.286 mg/kg parenterally.

8. The method of any of claims 1 to 3, **characterized in that** the ACE inhibitor is ramipril and the ANG II antagonist is telmisartan.

9. Pharmaceutical composition for the treatment of the human or non-human mammalian body for treating the indications mentioned in claims 1 to 3 comprising an ANG II antagonist and an ACE inhibitor as a combined preparation for simultaneous, separate or sequential use in treatment of said indications, optionally together with one or more pharmaceutically acceptable diluents and/or carriers.

10. The pharmaceutical composition of claim 9, **characterized in that** the ACE inhibitor is selected from benazepril, captopril, ceronapril, enalapril, fosinopril, imidapril, lisinopril, mo-exipril, quinapril, ramipril, trandolapril and perindopril in an amount of 1.25 mg to 100 mg and the ANG II antagonist is selected fom candesartan, eprosartan, irbesartan, losartan, telmisartan and valsartan, olmesartan, tasosartan in an amount of 20 to 100 mg in single dosage units.

11. The pharmaceutical composition of claim 9, **characterized in that** the ACE inhibitor is ramipril in an amount of 1.25 mg to 100 mg and the ANG II antagonist is selected from candesartan, eprosartan, irbesartan, losartan, telmisartan and valsartan in an amount of 20 mg to 100 mg in single dosage units.

12. The pharmaceutical composition of claim 9, **characterized in that** the ACE inhibitor is selected from benazepril, captopril, ceronapril, enalapril, fosinopril, imidapril, lisinopril, moexipril, quinapril, ramipril and trandolapril in an amount of 1.25 mg to 100 mg and the ANG II antagonist is telmisartan in an amount of 20 mg to 100 mg in single dosage units.

13. The pharmaceutical composition of claim 9, **characterized in that** the ACE inhibitor is selected from enalapril, lisinopril and ramipril in an amount of 1.25 mg to 100 mg and the ANG II antagonist is selected fom losartan and telmisartan in an amount of 20 mg to 100 mg in single dosage units.

14. The pharmaceutical composition of claim 9 or 10, **characterized in that** the ACE inhibitor is ramipril and the ANG II antagonist is telmisartan.

15. A pharmaceutical composition for treatment of elevated blood pressure or congestive heart failure in a mammal comprising an effective amount of the ACE inhibitor ramipril together with an effective amount of the ANG II antagonist telmisartan.

16. Use of an ANG II antagonist for manufacture of a pharmaceutical composition according to any of claims 9 to 14 for the treatment of the human or non-human mammalian body for treating the indications mentioned in claims 1 to 3 when used in combination with an ACE inhibitor.

17. Use of the ANG II antagonist telmisartan for manufacture of a pharmaceutical composition according to claim 15 for the treatment of the human or non-human mammalian body for treating elevated blood pressure or congestive heart failure when used in combination with the ACE inhibitor ramipril.
